# EUROPEAN PATENT APPLICATION

(11) **EP 3 991 560 A1**
(43) Date of publication of application: **04.05.2022**
(21) Application number: 21205569.3
(22) Date of filing: 29.10.2021
(51) Int. Cl.: A01N 31/08, A01N 37/06, A01N 37/10, A01N 37/36, A01N 59/20, A01P 1/00, A61L 2/23, A01N 25/34, A01N 25/12, A01N 25/30

(54) **STABLE ANHYDROUS DISINFECTANT CONCENTRATE FORMULATION AND METHOD OF MAKING THE SAME**

(30) Priority: 29.10.2020 US 202063106998 P
(71) Applicant: One Home Brands, Inc., New York, NY 10024-4926 (US)
(72) Inventor: NAQVI, Syed Humza, Walnut Creek, CA California 94597 (US); HAREWOOD, Patrick Shane, West Haven, CT Connecticut 06516 (US)
(74) Representative: Betten & Resch

(57) **Abstract**

The invention relates to stable, anhydrous concentrated disinfectant formulations.

## Description

### BACKGROUND

The majority of disinfectant products on the market are in liquid or gel forms and packaged in a plastic tube, bottle, spray bottle, or pump dispenser. The problem is the packaging. Single use plastic is everywhere and it is wreaking havoc on the environment. Only 9% of all plastic is actually recycled, and packaging generates the largest portion of municipal waste (~30%). Packaged products are inefficient for businesses and the people who buy them.

Removing the water from disinfectant formulations removes the need for single use plastic packaging and the waste that comes with it, such as packaging waste, product waste, and the waste of resources used to ship water.

Thus, a need exists for new stable formulations of disinfectants that meet the needs of consumers, while also reducing the amount of waste generated in their production and shipping.

### SUMMARY OF THE INVENTION

The invention relates to stable anhydrous disinfectant cleanser concentrate formulations. The stable anhydrous disinfectant cleanser concentrate formulations may be in a solid form such as a tablet, granulars, powder, sachet, or polymer membrane (PVA, PVP, HPMC, etc) form. The solid stable anhydrous disinfectant cleanser concentrate formulations comprise a disinfectant, an acidic cleaner, a pH control agent which can be a basic cleaner, and an oily soil remover (a surfactant). The solid stable anhydrous disinfectant cleanser concentrate formulation may further comprise a binder. The solid stable anhydrous disinfectant cleanser concentrate formulation may further comprise at least one natural and/or synthetic fragrance. The solid stable anhydrous disinfectant cleanser concentrate formulation may further comprise a dye or coloring agent. The solid stable anhydrous disinfectant cleanser concentrate formulation may comprise other ingredients to aid the disinfectant process.

The solid stable anhydrous disinfectant cleanser concentrate formulation may be used as a disinfectant spray tablet or sachet, disinfectant multi-purpose spray cleaner tablet or sachet, disinfectant bathroom spray cleaner tablet or sachet, toilet bowl cleaner tablet sachet or tablet, spray or gel, disinfectant hand soap sachet or tablet, disinfectant wipes sachet or tablet, drain opener tablet or sachet, disinfectant laundry tablet or sachet. When diluted by the end user, the solid stable anhydrous disinfectant cleanser concentrate formulation may be used to clean hands, bathroom surfaces, kitchen surfaces, glass, multi-surface, and any other areas including but not limited to daily shower room, toilet bowl, floor, etc.

### DETAILED DESCRIPTION OF THE INVENTION

This disclosure relates to solid stable anhydrous disinfectant cleanser concentrate formulations. The inventors have discovered a solid disinfectant formulation that is both good for the environment and effective for cleaning purpose. The advantages of this solid disinfectant formulation over the traditional liquid disinfectant cleansers include chemical stability, reduced packaging due to lack of water in the formulation, reduced shipping weight due to lack of water in the formulation, and convenience for the consumer.

Specifically, the solid stable anhydrous disinfectant cleanser concentrate formulations of this disclosure may include a disinfectant, an acidic cleaner, a pH control agent (e.g., a basic cleaner), an oily soil remover (e.g., a surfactant), and an optional ingredient selected from a binder and a preservative. The formulation may optionally include one or more fragrances, dyes, or coloring agents.

As used in this specification, the singular forms "a," "an," and "the" include plural referents unless the context clearly dictates otherwise. Thus, for example, reference to a "preservative" includes a single kind of preservative or two or more different kinds of preservative.

"About" as used herein means within an acceptable error range for the particular value as determined by one of ordinary skill in the art, which will depend in part on how the value is measured or determined, (*i.e.*, the limitations of the measurement system). For example, "about" can mean within 1 or more than 1 standard deviations, per practice in the art. Where particular values are described in the application and claims, unless otherwise stated, the term "about" means within an acceptable error range for the particular value. The term "about" when qualifying a value of a stated item, number, percentage, or term refers to a range of plus or minus ten percent of the value of the stated item, percentage, parameter, or term.

The term "oily soil remover" is used interchangeably with "surfactant."

The term "anhydrous" as used herein refers to a stable, anhydrous disinfectant cleanser concentrate formulation comprising less than about 5%, 4%, 3%, 2% or 1% by weight of water based on the weight of the concentrate.

The term "substantially fatty acid-free" as used herein refers to a solid stable, anhydrous cleanser concentrate formulation comprising less than 10%, 9%, 8%, 7%, 6%, 5%, 4%, 3%, 2%, or 1% by weight of a fatty acid (or salt thereof) based on the weight of the concentrate, or comprising a fatty acid (or salt thereof) in an amount less than the amount used in a hand soap bar.

The term "substantially animal fat free" as used herein refers to a solid stable, anhydrous disinfectant cleanser concentrate formulation comprising less than 10%, 9%, 8%, 7%, 6%, 5%, 4%, 3%, 2%, or 1% by weight of an animal fat (such as tallow) (or salt thereof) based on the weight of the concentrate, or comprising or comprising a an animal fat (or salt thereof) in an amount less than the amount used in a hand soap bar.

Some of the ingredients may have multiple functions. However, when two or more ingredients defined based on their functions are included in a formulation disclosed herein, the ingredients differ from each other in terms of their chemical structure. For example citric acid can be a water softening agent and an acidic cleaner as well, but when both water softening agent and acidic cleaner are used in the description of the formulation, they intend to refer to different ingredients in terms of the chemical structure.

The term "comprising" includes the embodiments of "consisting of' or "consisting essentially of."

The solid stable anhydrous disinfectant cleanser concentrate formulation includes a disinfectant to kill microorganisms, such as bacteria or viruses. The disinfectant may by thymol. Thymol is a component of thyme oil and is similar to the plant extract. Thyme oil is a minimal risk pesticide and has low toxicity. Thyme oil may be incorporated as part of an essential oil or fragrance. Thymol works as a disinfectant by inducing instability in a microorganism's membrane, causing cell content leakage and death of the microorganism.

The amount of thymol in the solid stable anhydrous disinfectant cleanser concentrate formulation may range from about 1% to about 40% by weight. The amount of disinfectant per formulation may be about 1%, about 5%, about 10%, about 15%, about 20%, about 25%, about 30%, about 40%, about 5% to about 40%, about 10% to about 35%, about 10% to about 30%, about 15% to about 25%, about 20% to about 25% by weight of the formulation.

After the solid stable anhydrous disinfectant cleanser concentrate formulation is diluted in water, the concentration of thymol will be reduced to a lower amount, such as about 0.05% to about 0.30% by weight. For example, if the thymol concentration of 5% by weight in a 8.0 gram solid stable anhydrous disinfectant cleanser concentrate tablet (about 0.40 grams of thymol) is diluted in 24 ounces of water (which is about 710 grams of water), the thymol concentration is reduced to about 0.056% by weight. As another example, if the thymol concentration of 22.5% by weight in a 8.0 gram solid stable anhydrous disinfectant cleanser concentrate formulation (about 1.64 grams of thymol) is diluted in 24 ounces of water (which is about 710 grams of water), the thymol concentration is reduced to about 0.23% by weight.

In some examples, the solid stable anhydrous disinfectant cleanser concentrate formulation may also include copper sulfate (bluestone). The copper sulfate may help the thymol work properly as a disinfectant. Copper sulfate has properties as disinfectant. The amount of copper sulfate in the solid stable anhydrous disinfectant cleanser concentrate formulation may range from about 0% to about 20% by weight, such as about 0%, about 5%, about 10%, by weight of the formulation. When included, the copper sulfate may be in a ratio of about 2:1 with the thymol (copper sulfate : thymol). After the solid stable anhydrous disinfectant cleanser concentrate formulation is diluted in water, the concentration of copper sulfate will be reduced to a lower amount, such as reduced to 0.10%.

In some examples, the solid stable anhydrous disinfectant cleanser concentrate formulation may include sorbitan caprylate. The sorbitan caprylate may help the thymol work properly as a disinfectant. The sorbitan caprylate may help disturb the microorganism cell walls, which allows other ingredients to enter the microorganism and kill it. The sorbitan caprylate may also act as a preservative booster by helping to kill microorganisms that would harm the formulation. The amount of sorbitan caprylate in the solid stable anhydrous disinfectant cleanser concentrate formulation may range from about 1% to about 5% by weight, such as about 0.1%, about 1%, about 0.5%, by weight of the formulation.

The amount of acidic cleaner in the solid stable anhydrous disinfectant cleanser concentrate formulation may range from about 1% to about 50% by weight, based on the weight of the formulation. The amount of acidic cleaner per tablet may be about 1%, about 5%, about 10%, about 15%, about 20%, about 25%, about 30%, about 40%, about 50%, about 5% to about 50%, about 10% to about 45%, about 15% to about 40%, about 20% to about 35%, about 30% to about 35%, by weight of the formulation. The acidic cleaner may be citric acid. The citric acid may be a particular type of citric acid that is coated. The coated citric acid may aid in stability of the formulation. The coated citric acid may be coated so it will not react to base of the formulation, which may make it more stable tablet formula in humid environments. For example, the coated citric acid may be Jungbunzlauer Citrocoat N10, which is 90% citric acid and 10% sodium citrate. Other possible coated citric acids may include Citrocoat F 5000, which is 97.5% citric acid and 2.5% sodium citrate or Citrocoat DC, which is citric acid coated with maltodextrin. The acidic cleaner may also be malic acid, fumaric acid, acetic acid, sodium acetate (salt), and/or glycolic acid.

The solid stable anhydrous disinfectant cleanser concentrate formulation may contain a pH control agent in an amount sufficient to adjust the pH when dissolved in water from about 2.0 to about 12.5, from about 5.0 to about 6.0, from about 3.5 to about 4.5, from about 4.0 to about 5.0, from about 4.5 to about 5.5, or from about 9.5 to about 10.5. The pH of the dissolved tablet in water may be about 2.0, about 2.5, about 3.0, about 3.5, about 4.0, about 4.5, about 5.0, about 5.5, about 2.0 to about 5.5, about 7.5, about 8.0, about 8.5, about 9.0, about 10.0, about 10.5, about 11.0, about 11.5, about 12.0, about 12.5, or about 7.5 to about 12.5. The pH control agent may be any agent sufficient to raise or lower the pH of the tablet when dissolved in water, which includes a basic cleaner and an acidic cleaner, such as sodium carbonate, sodium bicarbonate, citric acid, or malic acid. When both an acidic cleaner and a pH control agent are contained in the solid formulation, the acidic cleaner differs from the pH control agent. An acidic pH of the solid stable anhydrous disinfectant cleanser concentrate formulation may also help act as a disinfectant by helping to kill microorganisms.

When the pH control agent is a basic cleaner, the amount of the basic cleaner may range from about 5 % to about 60%, from about 5 % to about 30 %, from about 10% to about 30%, from about 10% to about 25%, from about 5% to about 10%, from about 40% to about 60%, or from about 35% to about 45%, by weight, based on the weight of the formulation. The basic cleaner may be sodium carbonate, sodium bicarbonate and/or any other alkali carbonates.

The acidic cleaner and basic cleaner together are referred as effervescent ingredients. In one aspect, the solid stable anhydrous disinfectant cleanser concentrate formulation can comprise the effervescent ingredients in an amount ranging from about 30% to about 80 or from about 50% to about 55% by weight. The effervescent ingredients (e.g, the acidic and basic cleaner) can facilitate homogeneous distribution and dissolution of the surfactant into water before use.

The amount of a binder-in the solid stable, anhydrous disinfectant cleanser concentrate formulation may range from about 0 to about 50% such as about 1% to about 20%, less than about 5%, from about 0% to about 5%, from about 3% to about 7%, from about 4% to about 8%, by weight, based on the weight of the formulation. The amount of binder may be about 1%, about 1.5%, about 2.0%, about 2.5%, about 3.0%, about 3.5%, about 4.0%, about 4.5%, about 5.0%, about 5.5%, about 6.0%, about 6.5%, about 7.0%, about 7.5%, about 8.0%, about 8.5%, about 9.0%, about 9.5%, about 10.0%, about 2% to about 10.0%, about 3% to about 7%, about 1 % to about 10%, or about 4% to about 8% by weight. The amount of binding agent in the solid stable, anhydrous cleanser concentrate formulation may range from about 1% to about 20% by weight, based on the weight of the formulation. The amount of binding agent is suitable to form a tablet and may be about 1 %, about 2%, about 3%, about 4%, about 5%, about 6%, about 7%, about 8%, about 9%, about 10%, about 12%, about 15%, about 18%, about 20%, about 3% to about 7%, about 4% to about 8%, about 5% to about 10%, or about 10% to about 20%. The solid stable, anhydrous cleanser concentrate formulation may contain one or more binders selected from the group consisting of hydrated dextrate, polyethylene glycol (e.g., polyethylene glycol 8000), dextrose, microcrystalline cellulose, and maltodextrin, pullulan, starch, polyvinylpyrrolidone.

The amount of oily soil remover (a surfactant) in the solid stable, anhydrous cleanser concentrate formulation may range from 0.01 % to about 40% such as from about 1 % to about 20%, from about 2% to about 15%, from about 8% to about 12%, from about 1% to about 15%, from about 3% to about 7%, from about 6 % to about 20%, from about 16% to about 20%, or from about 10% to 14% by weight, based on the weight of the formulation. The amount of oily soil remover may be about 1 %, about 2%, about 3%, about 4%, about 5%, about 6%, about 7%, about 8%, about 9%, about 10%, about 12%, about 15%, about 18%, about 20%, about 16% to about 20%, about 3% to about 7%, about 4% to about 8%, about 8% to about 12%, about 5% to about 10%, about 10% to about 15%, or about 10% to about 20%. The oily soil remover (i.e. surfactant) can be natural or synthetic surfactants, such an anionic, nonionic, amphoteric, zwitterioinic, or cationic surfactants, such as anionic and non-ionic surfactants, further such as a surfactant selected from sodium coco sulfate, ethoxylated alcohols (such as ethoxylated alcohol C(10-12)-C(14-16) with 4-8 moles ethoxylation, for example Clariant Genapol LA 060 (ethoxylated alcohol C12-C16) w/ 6 moles ethoxylation, ethoxylated alcohols C8-C10 6-8 moles of EO, etc.), sodium lauryl sulfate, sorbitan caprylate, cationic quaternary ammonium, and alkyl polyglucosides (such as lauryl glucoside, caprylyl / myristyl glucoside, caprylyl / decyl Glucoside).

The solid stable anhydrous disinfectant cleanser concentrate formulation may include a preservative. The amount of the preservative in the solid stable, anhydrous cleanser concentrate formulation may range from about 5 % to about 40%, from 5% to about 30 %, from about 10% to about 30%, from about 10% to about 25%, or from about 10% to about 20%, by weight, based on the weight of the formulation. The amount of the preservative may be about 5%, about 7%, about 9%, about 11%, about 13%, about 15%,about 17%, about 19%, about 21%, about 23%, about 25%, about 27%, about 29%, about 31%, about 33%, about 35%, about 37%, about 39%, or about 40%. The preservative may be sodium benzoate, potassium sorbate, gluconolactone, and/or biocideal preservatives. In some examples, sodium benzoate may also work as process aid for processing the powder to make the formulation into tablets.

The amount of the preservative booster in the solid stable anhydrous disinfectant cleanser concentrate formulation may range from about 0.1 % to about 15%, from about 0.5 % to about 10 %, from about 1 % to about 10 %, or from about 1% to about 5%, by weight, based on the weight of the formulation. The amount of the preservative booster may be about 0.1%, about 0.2 %, about 0.3%, about 0.4%, about 0.5%, about 1%, about 2%, about 3%, about 4%, about 5%, about 6%, about 7%, about 8%, about 9%, or about 10%. The preservative booster may be a sorbate such as potassium sorbate.

The solid stable anhydrous disinfectant cleanser concentrate formulation may contain one or more fragrances, such as natural fragrances (e.g., essential oils) and/or synthetic fragrances and perfumes in the form of oils, crystals, powders, granules, and encapsulations. The thymol in the formulation may partially come from thyme oil added as a fragrance.

The solid stable anhydrous disinfectant cleanser concentrate formulation may contain one or more dyes or coloring agents, such as Food, Drug and Cosmetic (FD&C) approved dyes and colorants.

In some embodiments the solid stable anhydrous disinfectant cleanser concentrate formulation may include silica as a flow aid for the formation of free-flowing powder. Free-flowing powder is subsequently converted into tablets.

In some embodiments, the solid stable anhydrous disinfectant cleanser concentrate formulation does not include silica. The precursor such as free-flowing powder can be formed by using solids, such as sodium carbonate and sodium benzoate, as the initial ingredients to absorb any potential liquids that can be used in the cleaner concentrate formulation.

In some embodiments, the solid stable anhydrous disinfectant cleanser concentrate formulation may be formed into a tablet that is dilutable in water. In other embodiments, the solid stable anhydrous disinfectant cleanser concentrate formulation may be other forms, such as a sachet with powder or crystals, powder with membrane (PVA, HPMC, PVP, pullulan, etc), wet granulation, spray dried powder in sachet or tablet, liquid concentrate to dilute, pod type with powder or liquid concentrate, or gel concentrate.

When the solid stable anhydrous disinfectant cleanser concentrate formulation is in the form of a tablet. The tablet can be in any size. For example, it may weigh from about 2.0 grams to about 18 grams, such as from about 4.5 grams to about 5.5 grams, such as from about 6.5 grams to about 7.1 grams, from about 6.0 grams and about 7.5 grams, from about 7.9 grams to about 8.5 grams, about 2.5 grams, about 3 grams, about 4 grams, about 5 grams, about 6 grams, about 7 grams, about 8 grams or more preferably about 9 grams. In a preferred embodiment, the tablets are round, however other geometric shapes are contemplated.

The tablet can be dissolved into appropriate amount of water before use. For example, the ratio of tablet to water can range from 5 - 12 grams of tablets in 24 - 34 ounces of water, or more preferably about 8.0 grams of tablets to 24 ounces of water, 9.33 grams of tablets to 28 ounces of water, 10.66 grams of tablets to 32 ounces of water, or 11.33 grams of tablets to 34 ounces of water.

In each of the preceding embodiments, the solid stable anhydrous disinfectant cleanser concentrate formulation can be substantially fatty acid free and/or substantially animal fat free.

The solid stable anhydrous disinfectant cleanser concentrate formulations may be a disinfectant spray tablet or sachet, disinfectant multi-purpose spray cleaner tablet or sachet, disinfectant bathroom spray cleaner tablet or sachet, toilet bowl cleaner tablet sachet or tablet, spray or gel, disinfectant hand soap sachet or tablet, disinfectant wipes sachet or tablet, drain opener tablet or sachet, disinfectant laundry tablet or sachet.

The solid stable anhydrous disinfectant cleanser concentrate formulations may be diluted by the end user at their home in order to create the final diluted formulation. The final diluted formulation may be used to clean hands, bathroom, glass, multi-surface, and any other areas including but not limited to daily shower room, toilet bowl, floor, etc.

### Methods for Preparing Stable Anhydrous Disinfectant Cleanser Tablets

The solid stable anhydrous disinfectant cleanser tablets can be prepared using any suitable method. Stable anhydrous disinfectant cleanser tablet can be prepared using direct compression or wet granulation process. The term direct compression (or direct compaction) is used to define the process by which tablets are compressed directly from powdered substance and suitable excipients into a firm compact without employing the process of granulation. Powder is blended homogeneously using a blender (Ribbon Blender, V-blender, paddle blender, drum mixing). The powder blender is then charged into the hopper of tablet press (such as a Stokes DS-3). Desire weight, compression ton, and hardness of tablet are set as the tablets get compressed and come out of the tablet press.

To avoid effervescence from happening during storage, the disinfectant cleanser formulation can be pressed to achieve a sufficient hardness and/or the formulation contains a desiccating agent such as hydrated silica or any other agent known to absorb moisture.

### Format

The stable anhydrous disinfectant cleanser concentrate formulations in powder form can also be diluted in water in a powder to water ratio of greater than or equal to1:1 (w/w) to form a paste before shipment. A method of using the paste comprises placing the paste on surface to be cleaned either directly or through a rag or sponge, optionally leaving overnight to soak, and rinsing the surface water.

The solid stable anhydrous disinfectant cleanser concentrate formulations described herein may be designed to be rinsed off, wiped, off, or left off for maximum cleaning efficiency.

### Methods of Using Stable Anhydrous Disinfectant Cleanser Tablets

In one aspect, disclosed is a method of using any of the tablets described herein including the steps of (1) filling a spray bottle or vessel with volume of 16-34 ounces with water, (2) adding the disinfectant cleaning tablet to the water-filled spray bottle, and (3) dissolving the tablet in water by no stirring or shaking required. In some embodiments, one or more disinfectant cleaning tablets may be added to the water-filled spray bottle. For example, two disinfectant cleaning tablets may be added to the spray bottle simultaneously or in a row before ultimately using the liquid solution for disinfectant cleaning purpose.

Each individual disinfectant cleanser tablet, when exposed to water, will dissolve into a liquid cleansing solution. Upon experiencing dissolution of the disinfectant cleanser tablet, the user may proceed with disinfectant cleaning or washing as usual. Individual tablets may be packaged together in suitable bulk quantities.

The disinfectant cleanser tablets may be stored in any suitable container, such as but not limited to paper, foil, plastic, glass, aluminum, ceramic, acrylic, or copolyester (Eastman Tritan) containers. The container may contain a desiccant. The container may be reusable and refilled with new tablets as needed.

### Exemplification

All the disclosed aspects of the stable anhydrous disinfectant cleanser concentrate formulations may be used with or applied to any of the examples listed.

### Example 1:

**Table 1**

| **Ingredient** | **Weight (%)** | **Weight (g)** |
|---|---|---|
| Sodium Carbonate (Base) | 18.00 | 1.44 |
| Fragrance | 1.50 | 0.12 |
| Sorbitan Caprylate (Nonionic Surfactant) | 0.50 | 0.04 |
| Sodium Benzoate (Preservative) | 14.00 | 1.12 |
| Potassium Sorbate (Preservative) | 4.00 | 0.32 |
| Hydrated Dextrate (Binder) | 5.00 | 0.40 |
| Sodium Lauryl Sulfate (Anionic Surfactant) | 6.00 | 0.48 |
| Thymol Crystals = >0.36gram/725g (Active Ingredient) | 5.00 | 0.40 |
| Copper Sulfate (bluestone) = 2X Thymol | 10.00 | 0.80 |
| Citric Acid (Citrocoat N10) (Acid) | 36.00 | 2.88 |
| Total | 100.00 | 8.00 |

Example 1 may be formed into a solid tablet weighing 8.0 grams. Example 1 may have a pH of about 3.0. Due to the relatively low pH, Example 1 may preferably be used as a bathroom cleaner and/or a tub and tile cleaner. Example 1 may have 0.05% thymol in the final diluted liquid disinfectant cleansing solution when diluted with about 24 ounces of water. Example 1 may include copper sulfate to aid the thymol working as a disinfectant.

Additional exemplary cleaner formulations with relatively low pH that may be used as a bathroom cleaner and/or a tub and tile cleaner are shown in Table 2.

| | **Low pH Formulation A** | | **Low pH Formulation B** | | **Low pH Formulation C** | |
|---|---|---|---|---|---|---|
| Ingredient | Weight (%) | Weight (g) | Weight (%) | Weight (g) | Weight (%) | Weight (g) |
| Sodium Carbonate (Base) | 20.00 | 1.60 | 18.50 | 1.48 | 18.00 | 1.44 |
| Fragrance | 2.00 | 0.16 | 2.00 | 0.16 | 2.00 | 0.16 |
| Sorbitan Capylate (Nonionic Surfactant - Fragrance emulsifier) | 0.50 | 0.04 | 0.50 | 0.04 | 0.50 | 0.04 |
| Sipernat 50 (Silica - drying agent) | 1.00 | 0.08 | 1.00 | 0.08 | 1.00 | 0.08 |
| Sodium Gluconate (chelting agent/ q.s agent / Filler) | 23.50 | 1.88 | 17.55 | 1.40 | 13.50 | 1.08 |
| Hydrated Dextrate (Binder) | 5.00 | 0.40 | 5.00 | 0.40 | 5.00 | 0.40 |
| Sodium Lauryl Sulfate (Anionic Surfactant - dirt, grime, and grease remover) | 5.00 | 0.40 | 5.00 | 0.40 | 5.00 | 0.40 |
| Sodium Coco Sulfate - granulated, (Anionic Surfactant - dirt, grime, and grease remover) | 4.00 | 0.32 | 4.00 | 0.32 | 4.00 | 0.32 |
| Thymol Crystals (Disinfectant Active agent) | 5.00 | 0.40 | 5.00 | 0.40 | 5.00 | 0.40 |
| Copper Sulfate (helps disinfect) | 10.00 | 0.80 | 10.00 | 0.80 | 10.00 | 0.80 |
| Coated Citric Acid (Citrocoat N10) | 24.00 | 1.92 | 31.45 | 2.52 | 36.00 | 2.88 |
| Total | 100.00 | 8.00 | 100.00 | 8.00 | 100.00 | 8.00 |
| Tablet Size | | 8.00 | | 8.00 | | 8.00 |
| pH | 4.3-4.6 | | 3.9-4.2 | | 3.0-3.3 | |
| Table 2 | | | | | | |

### Example 2:

**Table 3**

| **Ingredient** | **Weight (%)** | **Weight (g)** |
|---|---|---|
| Sodium Carbonate (Base) | 23.00 | 1.84 |
| Fragrance | 1.50 | 0.12 |
| Sorbitan Caprylate (Nonionic Surfactant) | 0.50 | 0.04 |
| Sodium Benzoate (Preservative) | 14.00 | 1.12 |
| Potassium Sorbate (Preservative) | 4.00 | 0.32 |
| Hydrated Dextrate (Binder) | 5.00 | 0.40 |
| Sodium Lauryl Sulfate (Anionic Surfactant) | 6.00 | 0.48 |
| Thymol Crystals = >0.36gram/725g (Active Ingredient) | 5.00 | 0.40 |
| Copper Sulfate (bluestone) = 2X Thymol | 10.00 | 0.80 |
| Citric Acid (Citrocoat N10) (Acid) | 31.00 | 2.48 |
| Total | 100.00 | 8.00 |

Example 2 may be formed into a solid tablet weighing 8.0 grams. Example 2 may have a pH of about 5-6. Due to the pH, Example 2 may be used as a cleaner for multiple surfaces. Example 2 may have 0.05% thymol in the final diluted liquid disinfectant cleansing solution when diluted with about 24 ounces of water. Example 2 may include copper sulfate to aid the thymol working as a disinfectant.

Additional exemplary cleaner formulations with moderate pH that may be used for multiple surfaces are shown in Table 4.

| | Moderate pH Formulation A | | Moderate pH Formulation B | | Moderate pH Formulation C | |
|---|---|---|---|---|---|---|
| Ingredient | Weight (%) | Weight (g) | Weight (%) | Weight (g) | Weight (%) | Weight (g) |
| Sodium Carbonate (Base) | 35.00 | 2.80 | 30.00 | 2.40 | 25.00 | 2.00 |
| Fragrance | 2.00 | 0.16 | 2.00 | 0.16 | 2.00 | 0.16 |
| Sorbitan Capylate (Nonionic Surfactant - Fragrance emulsifier) | 0.50 | 0.04 | 0.50 | 0.04 | 0.50 | 0.04 |
| Sipernat 50 (Silica - drying agent) | 1.00 | 0.08 | 1.00 | 0.08 | 1.00 | 0.08 |
| Sodium Gluconate (chelting agent/ q.s agent / Filler) | 7.50 | 0.60 | 12.50 | 1.00 | 17.50 | 1.40 |
| Hydrated Dextrate (Binder) | 5.00 | 0.40 | 5.00 | 0.40 | 5.00 | 0.40 |
| Sodium Lauryl Sulfate (Anionic Surfactant - dirt, grime, and grease remover) | 5.00 | 0.40 | 5.00 | 0.40 | 5.00 | 0.40 |
| Sodium Coco Sulfate - granulated, (Anionic Surfactant - dirt, grime, and grease remover) | 4.00 | 0.32 | 4.00 | 0.32 | 4.00 | 0.32 |
| Thymol Crystals (Disinfectant Active agent) | 5.00 | 0.40 | 5.00 | 0.40 | 5.00 | 0.40 |
| Copper Sulfate (helps disinfect) | 10.00 | 0.80 | 10.00 | 0.80 | 10.00 | 0.80 |
| Coated Citric Acid (Citrocoat N10) | 25.00 | 2.00 | 25.00 | 2.00 | 25.00 | 2.00 |
| Total | 100.00 | 8.00 | 100.00 | 8.00 | 100.00 | 8.00 |
| Tablet Size | | 8.00 | | 8.00 | | 8.00 |
| pH | 5.8-6.0 | | 5.3-5.5 | | 4.9-5.1 | |
| Table 4 | | | | | | |

### Example 3:

**Table 5**

| **Ingredient** | **Weight (%)** | **Weight (g)** |
|---|---|---|
| Sodium Carbonate (Dense) | 18.50 | 1.48 |
| Fragrance | 1.50 | 0.12 |
| Sorbitan Caprylate (Nonionic Surfactant) | 0.50 | 0.04 |
| Sodium Benzoate (Preservative) | 10.55 | 0.84 |
| Potassium Sorbate (Preservative) | 4.00 | 0.32 |
| Hydrated Dextrate (Binder) | 5.00 | 0.40 |
| Sodium Lauryl Sulfate (Anionic Surfactant) | 6.00 | 0.48 |
| Thymol Crystals = 1.80g / 725g of water > 0.23% Active Thymol | 22.50 | 1.80 |
| Citric Acid | 31.45 | 2.52 |
| Total | 100.00 | 8.00 |

Example 3 may be formed into a solid tablet weighing 8.0 grams. Example 3 may include a higher concentration of thymol than Examples 1 or 2. Example 3 may have about 0.23% thymol in the final diluted liquid disinfectant cleansing solution when diluted with about 725 grams of water. Example 3 may not include copper sulfate due to the relatively high concentration of thymol in Example 3. Instructions for dilution of an Example 3 tablet may state to use 24 ounces of water, which is about 710 grams, but if the end user dilutes an Example 3 tablet in more than 24 ounces of water, such as 725 grams of water, an Example 3 tablet will include enough thymol for the final diluted liquid disinfectant cleansing solution to be an effective disinfectant.

An additional exemplary cleaner formulation with a relatively higher concentration of thymol is shown in Table 6.

**Table 6**

| | **High Thymol Formulation A** | |
|---|---|---|
| Ingredient | Weight (%) | Weight (g) |
| Sodium Carbonate (Base) | 18.50 | 1.48 |
| Fragrance | 2.00 | 0.16 |
| Sorbitan Capylate (Nonionic Surfactant - Fragrance emulsifier) | 0.50 | 0.04 |
| Sipernat 50 (Silica - drying agent) | 1.00 | 0.08 |
| Sodium Gluconate (chelting agent/ q.s agent / Filler) | 11.13 | 0.89 |
| Hydrated Dextrate (Binder) | 5.00 | 0.40 |
| Sodium Lauryl Sulfate (Anionic Surfactant - dirt, grime, and grease remover) | 6.00 | 0.48 |
| Sodium Coco Sulfate - granulated, (Anionic Surfactant - dirt, grime, and grease remover) | 4.00 | 0.32 |
| Thymol Crystals (Disinfectant Active agent) | 20.42 | 1.63 |
| Copper Sulfate (helps disinfect) | 0.00 | 0.00 |
| Coated Citric Acid (Citrocoat N10) | 31.45 | 2.52 |
| Total | 100.00 | 8.00 |
| Tablet Size | | 8.00 |
| pH | 4.4 - 4.6 | |
| Notes: 1.633g Thymol / 710g (24 oz) of water = 0.23% Active | | |

The disclosed aspects of the stable anhydrous disinfectant cleanser concentrate formulations may also be used in disinfectant formulations that are not designed to be cleansers or that do not function as cleansers. The stable anhydrous disinfectant concentrate formulations may include relatively low amounts of surfactants and may have a safe pH range to allow use of the disinfectant as a spray disinfectant on any surface.

Exemplary stable anhydrous disinfectant concentrate formulations are shown in Table 7.

**Table 7**

| | **Disinfectant Formulation A** | | **Disinfectant Formulation B** | | **Disinfectant Formulation C** | |
|---|---|---|---|---|---|---|
| Ingredient | Weight (%) | Weight (g) | Weight (%) | Weight (g) | Weight (%) | Weight (g) |
| Sodium Carbonate (Base) | 35.00 | 2.80 | 30.00 | 2.40 | 25.00 | 2.00 |
| Fragrance | 2.00 | 0.16 | 2.00 | 0.16 | 2.00 | 0.16 |
| Sorbitan Capylate (Nonionic Surfactant - Fragrance emulsifier) | 0.50 | 0.04 | 0.50 | 0.04 | 0.50 | 0.04 |
| Sipernat 50 (Silica - drying agent) | 1.00 | 0.08 | 1.00 | 0.08 | 1.00 | 0.08 |
| Sodium Gluconate (chelting agent/ q.s agent / Filler) | 13.50 | 1.08 | 18.50 | 1.48 | 23.50 | 1.88 |
| Hydrated Dextrate (Binder) | 5.00 | 0.40 | 5.00 | 0.40 | 5.00 | 0.40 |
| Sodium Lauryl Sulfate (Anionic Surfactant - dirt, grime, and grease remover) | 3.00 | 0.24 | 3.00 | 0.24 | 3.00 | 0.24 |
| Sodium Coco Sulfate - granulated, (Anionic Surfactant - dirt, grime, and grease remover) | 0.00 | 0.00 | 0.00 | 0.00 | 0.00 | 0.00 |
| Thymol Crystals (Disinfectant Active agent) | 5.00 | 0.40 | 5.00 | 0.40 | 5.00 | 0.40 |
| Copper Sulfate (helps disinfect) | 10.00 | 0.80 | 10.00 | 0.80 | 10.00 | 0.80 |
| Coated Citric Acid (Citrocoat N10) | 25.00 | 2.00 | 25.00 | 2.00 | 25.00 | 2.00 |
| Total | 100.00 | 8.00 | 100.00 | 8.00 | 100.00 | 8.00 |
| Tablet Size | | 8.00 | | 8.00 | | 8.00 |
| pH | 5.8-6.0 | | 5.3-5.5 | | 4.9-5.1 | |

## Claims

1. A stable anhydrous disinfectant cleanser concentrate formulation in a solid form, comprising a disinfectant, an acidic cleaner, a basic cleaner, and a surfactant.

2. The stable anhydrous disinfectant cleanser concentrate formulation of claim 1, wherein the disinfectant is thymol.

3. The stable anhydrous disinfectant cleanser concentrate formulation of claim 2,
wherein the disinfectant is present in an amount from about 4% to about 25% by weight, based on the weight of the formulation; or
wherein the disinfectant is present in an amount from about 4% to about 6% by weight, based on the weight of the formulation.

4. The stable anhydrous disinfectant cleanser concentrate formulation of claim 2, wherein the disinfectant is present in an amount from about 20% to about 25% by weight, based on the weight of the formulation, and optionally the formulation does not include copper sulfate.

5. The stable anhydrous disinfectant cleanser concentrate formulation of claim 2, further comprising copper sulfate.

6. The stable anhydrous disinfectant cleanser concentrate formulation of claim 5,
wherein the copper sulfate is present in an amount from about 8% to about 12% by weight, based on the weight of the formulation; or
wherein the copper sulfate is present in an amount about twice the amount of thymol by weight.

7. The stable anhydrous disinfectant cleanser concentrate formulation of claim 2, further comprising a preservative, wherein the preservative is optionally selected from sodium benzoate and potassium sorbate.

8. The stable anhydrous disinfectant cleanser concentrate formulation of claim 2, further comprising sorbitan caprylate, wherein the sorbitan caprylate is optionally present in an amount from about 0.1% to about 1% by weight, based on the weight of the formulation.

9. The stable anhydrous disinfectant cleanser concentrate formulation of claim 1,
wherein the acidic cleaner is selected from citric acid, malic acid, fumaric acid, acetic acid, sodium acetate, and glycolic acid; or
wherein the acidic cleaner is a coated citric acid that is designed not to react with the basic cleaner in the formulation; or
wherein the basic cleaner is selected from sodium carbonate, sodium bicarbonate and any other alkali carbonates; or
wherein the surfactant comprises an anionic and/or nonionic surfactant; or
wherein the formulation further comprises a binding agent; or
wherein the formulation is substantially fatty acid free and/or substantially animal fat free; or
wherein at least one of the acidic cleaner and the basic cleaner is present in an amount greater than that of the surfactant; or
wherein the acidic cleaner and the basic cleaner together are present in an amount greater than that of the surfactant; or
wherein the formulation is inserted in a wipe; or
wherein the formulation is in the form of a tablet and the tablet is optionally not tacky; or
wherein the formulation is in the form of powder.

10. A method of preparing a tablet, comprising blending homogeneously the ingredients of claim 1 to form a mixture and compressing the mixture to form the tablet.

11. A method of using the stable anhydrous disinfectant cleanser concentrate formulation of claim 1 in the form of a tablet comprising (1) filling a spray bottle or vessel with water, (2) adding the tablet to the water-filled spray bottle or vessel, and (3) dissolving the tablet in appropriate amount of water.

12. The method of claim 11,
wherein the method further comprises applying the resulting solution to a surface to be cleaned; and optionally
wherein the surface to be cleaned is a hand or a surface in a bathroom or kitchen.

13. A method of using the stable anhydrous disinfectant cleanser concentrate formulation of claim 1 in the form of a powder comprising diluting the powder in water at a powder to water ratio of greater than or equal to 1:1 (w/w) to form a paste, placing the paste on a surface to be cleaned either directly or through a rag or sponge, and rinsing the surface with water.

14. A stable anhydrous disinfectant concentrate formulation in a solid form, comprising a disinfectant, an acidic cleaner, a basic cleaner, and a surfactant.

15. The stable anhydrous disinfectant concentrate formulation of claim 14, wherein the disinfectant is thymol.
